# DEMANDE DE BREVET EUROPEEN

(11) **EP 3 569 203 A1**
(43) Date de publication de la demande: **20.11.2019**
(21) Numéro de dépôt: 18020212.9
(22) Date de dépôt: 16.05.2018
(51) Int. Cl.: A61F 5/41

(54) **MÉTHODE ET APPAREILLAGE POUR LE TRAITEMENT DU DYSFONCTIONNEMENT ÉRECTILE**

(71) Demandeur: Remacle, Roger, 1380 Lasne (BE)
(72) Inventeur: Remacle, Roger, 1380 Lasne (BE)

(57) **Abrégé**

Appareil, ou constricteur pelvien, et méthode pour traiter le dysfonctionnement érectile qui se compose d'une seule pièce qui se place directement sur le corps et qui exerce une compression aussi bien ventrale que dorsale sur les nerfs et veines vidangeant le pénis.

## Description

La présente invention se rapporte à un appareillage simple mais efficace ainsi qu'à son l'utilisation pour le traitement des dysfonctionnements érectiles. En particulier, l'invention se rapporte à un appareillage constitué d'une seule pièce ou ceinture, adaptable à toutes les morphologies et qui stimule simultanément toutes les zones sensibles du bassin.

Les dysfonctionnements érectiles sont fréquemment rencontrés et peuvent atteindre
des valeurs de l'ordre de 40 à 50 % pour des hommes âgés de plus de quarante ans ; ces valeurs sont encore plus importantes si l'on se restreint à une population masculine atteinte du cancer de la prostate et qui ont subi une prostatectomie ou même d'insuffisance rénale.

Le mécanisme physiologique de l'érection est lié à un phénomène hémodynamique
qui est l'augmentation des flux artériels couplée à une réduction massive du drainage veineux. Ces phénomènes complexes, sous contrôle neurologique modulé par les sécrétions hormonales androgéniques, font intervenir des facteurs vasculaires et tissulaires ainsi que de nombreux transmetteurs dont certains ne sont pas encore totalement identifiés. Mécaniquement parlant, l'érection comporte trois phases : le remplissage qui est l'apport artériel, ensuite l'augmentation de volume de la verge par reflexe nerveux, ce qui enclenche le gonflement des cellules éponges et le durcissement des parois de la verge ; et enfin troisième phase, la fuite veineuse, qui est contrôlée et qui renouvelle le sang emprisonné et évite la nécrose.

Dans ce mécanisme, les tissus caverneux jouent un rôle essentiel ; en effet, l'érection ne résulte pas seulement d'une vasodilatation artérielle suivie d'un remplissage des corps caverneux, mais aussi d'une relaxation des fibres musculaires lisses des corps caverneux permettant l'aspiration du sang à l'intérieur des corps caverneux et une compression veineuse par écrasement des veines émissaires contre l'albuginée.

Des traitements du dysfonctionnement érectile sont largement décrits dans la littérature, allant des simples moyens mécaniques, vibratoires, destinés à stimuler les nerfs reliés au pénis, aux autres traitements médicamenteux ; ils sont spécifiques pour palier les dysfonctionnements des différentes phases de l'érection ; tout d'abord l'arrivée du sang est réglée par une amélioration du système artériel, tandis que le remplissage est résolu différents modes d'administration de principes actifs comme le traitement par injection intra caverneuse d'agents vaso-actifs comme des compositions comprenant de l'alprostadil et/ou de l'acide benzylique ; cependant, on reconnaît que dans plus de 50% des cas, les patients ont arrêté ce type de traitement du fait de difficultés psychologiques ou d'effets secondaires désagréables comme les nausées, les vomissements ou la tachycardie. Ces mêmes effets secondaires apparaissent également lors de la prise de médicaments ayant les mêmes fonctions curatives, comme les médicaments dont le principe actif est le tadalafil.

A côté de ces moyens médicamenteux il existe aussi des moyens plus invasifs comme
le remplacement de deux des trois corps caverneux par des enveloppes qui se remplissent d'un liquide contenu dans un réservoir fixé près de la vessie grâce à une pompe manuelle sertie dans les bourses, mais cette solution présente comme inconvénient majeur le risque d'infection tout en étant trop radicale.

Parallèlement à ces traitements médicamenteux ou chirurgicaux, on peut citer de nombreux moyens mécaniques, destinés notamment à stimuler les nerfs qui sont reliés directement ou indirectement au pénis. Ces moyens mécaniques comprennent des systèmes complexes qui doivent influer sur cette stimulation, comme les vibrations initiées électriquement ou mécaniquement, des systèmes qui assurent un massage avec compression des zones appropriées ou des nerfs qui conduisent au pénis.

Il est évident que ces moyens sont supportés à l'aide de plusieurs ceintures placées autour du corps.

Cependant, ces systèmes qui sont notamment décrits dans les brevets US 4759377, US 20100069708 ou CN 2376279 n'apportent pas de solutions simples à mettre en oeuvre.

Un des inconvénients majeurs de plusieurs ceintures connectées entre elles réside notamment dans la présence nécessaire de boucles de raccord qui sont très gênantes.

On peut également citer les brevets US8615815 et US 2009/0178182 qui décrivent des systèmes adaptés à la fois au traitement des hernies ainsi qu'à l'amélioration des performances sexuelles en particulier en apportant un moyen pour maintenir l'érection ; même si le but recherché dans ces deux brevets est similaire au nôtre, le moyen pour y parvenir pose question car la pression exercée pour maintenir la hernie en place est une pression exercée en continu et d'intensité faible car il est bien connu que l'on ne peut exercer une forte pression durant longtemps, avec le risque de nécrose, ce qui dès lors ne peut convenir pour traiter le maintien de l'érection., et par voie de conséquence, on ne peut traiter à la fois ou de la même manière la hernie et le dysfonctionnement érectile.

Comme on peut le voir à l'analyse de l'état de la technique, pour ce qui concerne les fuites veineuses qui entrainent un dysfonctionnement érectile il n'existe à l'heure actuelle aucun remède spécifique ni chirurgical ni médicamenteux ni mécanique.

Il existe donc un besoin pour la mise en oeuvre d'un système pour traiter le dysfonctionnement érectile, qui soit non seulement efficace, et qui stimule la majorité des veines responsables du dysfonctionnement érectile, mais aisé à mettre en oeuvre, discret, non douloureux, ni physiquement, ni psychologiquement et adaptables à toutes les morphologies et qui stimule les zones sensibles du bassin et qui soit confortable pour la(e) partenaire.

La présente invention a donc pour objet un appareil, ou constricteur pelvien, simple et discret constitué essentiellement en une seule pièce enfilable directement sur le corps et constitué d'un matériau ayant une texture élastique.

Un autre objet de l'invention réside en ce que le constricteur pelvien soit conçu pour mouler parfaitement la zone du corps comprise entre les cuisses, autour du sexe et allant jusqu'à la taille.

Un autre objet de l'invention réside en un constricteur pelvien qui enserre la base du pénis et du scrotum tout en laissant libre le pénis lui-même ainsi que les bourses.

Un autre objet de l'invention consiste à concentrer la pression en certains endroits.

Un autre objet de l'invention consiste en une méthode pour traiter les dysfonctionnements érectiles par utilisation du constricteur pelvien de l'invention.

Un autre objet de l'invention consiste en une méthode simple et aisément visible par l'utilisateur pour mesurer la pression exercée .

La Demanderesse a maintenant trouvé un appareil pour traiter le dysfonctionnement érectile, qui permette de remédier aux inconvénients des appareils antérieurs tels que décrits ci-dessus, et qui rencontre les objectifs souhaités, et qui ne soit pas lié à la prise conjointe de médicament.

L'appareil de l'invention se compose essentiellement d'une seule pièce en une matière ayant une texture élastique, qui se place directement sur le corps, de la même manière qu'un slip ou un maillot de bain.

L'appareil de l'invention est conçu pour mouler parfaitement la zone comprise entre les cuisses, autour du sexe et allant jusqu'à la taille avec une adhésion optimum sur les hanches. Il est conçu d'une manière telle qu'il enserre la base du pénis et du scrotum laissant ainsi libres le pénis lui-même ainsi que les bourses ; en particulier pour obtenir un effet significatif sur le dysfonctionnement érectile dû particulièrement aux fuites veineuses, la Demanderesse a maintenant trouvé qu'il est nécessaire d'exercer une pression suffisamment forte pour que les muscles pelviens soient poussés vers le haut et par effet domino compriment à leur tour les organes du bas ventre et que le tout comprime les deux bulbes caverneux intérieurs. La Demanderesse a maintenant trouvé qu'en exerçant cette compression dirigée du bas vers le haut et ce pendant un temps suffisant de manière à maintenir l'érection durant l'acte sexuel, on pouvait comprimer les veines d'évacuation des trois corps caverneux et ainsi remédier aux fuites veineuses.

Afin que la compression s'exerce de bas en haut, on prévoit d'utiliser un tissu suffisamment élastique plus à même d'exercer ce type de pression et permettre de juguler la fuite veineuse. Cette pression est notamment assurée par les boutons de fermeture par pression fixés sur les brins de l'appareil et résulte aussi du matériau utilisé pour l'appareil qui doit répondre aux propriétés physiques décrites comme la résistance à la traction et l'allongement à la rupture.

Il est bien entendu que cette pression peut varier, en fonction des besoins, et en règlant la tension exercée par les boutons de pression.

Afin que la pression soit apposée à certains endroits, l'invention comporte deux plots qui concentrent la pression. Le premier autour de la base de la verge, riche en veines qui évacuent une partie du sang des corps caverneux et un plot sous la base de la verge qui dirige la pression vers la cavité abdominale pour atteindre l'effet domino ; qui consiste à exercer une pression sur les muscles pelviens - pression transmise à l'intérieur de la cavité abdominale et qui comprime les bulbes caverneux dont la partie intérieure se prolongent presque jusqu'à l'anus.

Ces plots sont complémentaires à la stimulation simultanée de toutes les zones sensibles du bassin.

Afin que la pression exercée soit suffisante pour obtenir cet effet, l'appareil de l'invention comporte un système dynamométrique intégré qui évalue la pression exercée .

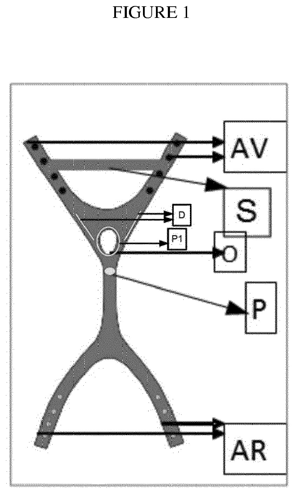

L'appareil de l'invention est également décrit au moyen de la Figure 1 qui donne une représentation schématique de l'appareil de l'invention qui comprend une partie ventrale V percée d'une ouverture ovale O, d'une bande abdominale de maintien S ainsi qu'une bande périnéale P ; il comprend également deux brins arrière AR ainsi que deux brins avant AV ; les brins comportent à leurs extrémités des boutons à fermeture par pression ; selon la Figure 1 la partie ventrale V comporte des reliefs P, P1 et D. Ces reliefs sont des plots d'ajustement de la pression. Ils sont situés autour et sous l'ouverture O .

Deux traits en reliefs D, longs de 10.0 cm, positionnés à gauche et à droite de l'ouverture ovale O, constituent le système dynamométrique.

Le terme élastique de la pièce élastique du système de l'invention, qu'elle soit sous forme de ceinture ou de slip ou de maillot de bain, signifie que le tissu ou le matériau qui la constitue a des propriétés élastiques ; en d'autres termes qu'elle peut subir un allongement,
par exemple en s'adaptant à la forme du corps, et revenir à sa position initiale ensuite.

La pièce élastique de l'invention, qu'elle soit sous forme de ceinture ou de slip, est également décrite à l'aide des dessins annexés qui représentent une vue schématique du constricteur pelvien.

Comme on peut le constater, elle est d'une seule pièce, de la forme adéquate pour épouser le bas du ventre, en parfaite adhésion sur les hanches et percée d'un trou à l'endroit du sexe.

A titre d'exemple de matériau dont la texture élastique convient pour le constricteur pelvien de l'invention, on peut notamment citer un matériau similaire à une chambre à air d'un pneu de camion. Cependant un matériau similaire et convenant pour le dispositif de l'invention peut se préparer au départ d'une résine réticulable ayant une viscosité de l'ordre de 15.000 à 20.000mPa s et dont le produit final a une densité comprise entre 1,1 et 1,2 ; une dureté Shore A de 35 à 40 ; une résistance à la traction de 5,0 à 7,5 N/mm2 ; un allongement à la rupture compris entre 400 et 650% et une résistance au déchirement > 30 N/mm. A titre d'exemple on peut également citer un tissu de coton renforcé avec de la matière élastique, bien connu en matière de textile, ou les tissus pour les maillots de bain mais toujours renforcés avec de la matière élastique.

Selon la présente invention on peut encore utiliser un tissu en polyester intégré dans une trame de silicone.

Le plot P constitué du même matériau élastique que l'invention est de forme ovale ; situé sous la base de la verge, il a la forme d'un demi oeuf ; il a une longueur de 7cm , une largueur de 4 cm et une hauteur de 1cm.

Le plot P1 est constitué du même matériau élastique que l'invention, entoure complètement la base de la verge. Sa forme est celle d'un anneau hémisphérique de 6 cm de diamètre de largeur 0,5 cm et d'épaisseur de 0,5 cm.

L'échelle du système dynamométrique est un trait en relief qui mesure 10 cm. Sous la tension, il s'allonge et peut atteindre des valeurs supérieures à 10 cm et atteindre des valeurs de 16 cm. L'évaluation de la pression se fait au moyen d'une réglette (fournie) qui mesure l'élongation des traits en relief. Le minimum à atteindre est de 12 cm.

La mise en oeuvre de l'appareil de l'invention découle directement de sa composition élastique qui permet de comprimer le bas ventre, le périnée, la base du pénis et les cuisses,
le tout simultanément, et la pression exercée étant dirigée de bas en haut.

La seule compression exercée par l'appareil de l'invention sur les zones à stimuler, comme les nerfs ou les veines atteignant le pénis, ou même les méridiens qui les desservent, est suffisante pour palier un dysfonctionnement érectile avec efficacité.

Un des avantages du constricteur pelvien de l'invention est qu'il est constitué d'une et unique pièce qui exerce une compression aussi bien ventrale, périnéale que dorsale, dirigée de bas en haut sur tous les nerfs et veines conduisant au pénis et ce sans aide aucune, ni électrique ni mécanique.

Il est entendu que d'autres applications de ce constricteur peuvent être envisagées sans se départir de la présente invention.

## Revendications

1. Appareil pour traiter le dysfonctionnement érectile, **caractérisé en ce qu'**il se compose essentiellement d'une seule pièce élastique qui se place directement sur le corps, (de la même manière qu'un slip ou un maillot de bain) et qui moule parfaitement la zone comprise entre les cuisses, autour du sexe et allant lui-même ainsi que les bourses t jusqu'à la taille, tout en enserrant la base du pénis et du scrotum laissant ainsi libres le pénis et en exerçant un compression conséquente dirigée de bas en haut.

2. Appareil selon la revendication 1 **caractérisé en ce que** la pièce élastique se compose d'un tissu de coton renforcé avec une matière élastique choisie parmi les textiles élastiques bien connus.
Appareil selon la revendication 1 **caractérisé en ce que** la pièce élastique se compose d'un tissu polyester intégré dans une trame en silicone.

3. Appareil selon la revendication 3 **caractérisé en ce que** la pièce élastique est préparée à partir d'une résine réticulable ayant une viscosité de l'ordre de 15.000 à 20.000mPa s et dont le produit final a une densité comprise entre 1,1 et 1,2, une dureté Shore A de 35 à 40 , une résistance à la traction de 5,0 à 7,5 N/mm2 , un allongement à la rupture compris entre 400 et 650% et une résistance au déchirement > 30 N/mm.

4. Méthode pour remédier au dysfonctionnement érectile, **caractérisé en ce qu'**elle consiste à appliquer une compression dirigée de bas en haut simultanément sur le bas ventre, le périnée, la base du pénis et les cuisses, au moyen de la pièce élastique définie dans les revendications 1 à 4.

5. Méthode selon la revendication 5 **caractérisé en ce que** la compression est assurée par les boutons de fermeture par pression fixés sur les brins (AV) et (AR) de l'appareil.

6. Utilisation du constricteur pelvien décrit dans les revendications 1 à 4 pour remédier aux dysfonctionnements érectiles.
